# EUROPEAN PATENT APPLICATION

(11) **EP 1 992 295 A1**
(43) Date of publication of application: **19.11.2008**
(21) Application number: 08251601.4
(22) Date of filing: 01.05.2008
(51) Int. Cl.: A61B 17/064

(54) **Single fire tacker instrument**

(30) Priority: 07.05.2007 US 800903
(71) Applicant: Tyco Healthcare Group, LP, North Haven, CT 06473 (US)
(72) Inventor: Zergiebel, Earl M., Guilford, CT 06430 (US); Addi, Ernest, Middletown, CT 06457 (US); Aranyi, Ernie, Easton, CT 06612 (US)
(74) Representative: Alcock, David

(57) **Abstract**

A single fire tacker instrument is provided for installing a fastener through a prosthetic mesh and into tissue. The single fire tacker instrument includes a handle assembly and an elongated tacker assembly extending distally from the handle assembly. The elongated tacker assembly includes an inner tube for mounting the elongated tacker assembly to the handle assembly. The elongated tacker assembly includes a drive rod and a driver for rotating the faster into tissue. A spring clip is provided about the driver to releasably retain the fastener on the driver. The elongated tacker assembly and additionally includes a spring biased outer tube mounted for movement relative to the handle assembly. The outer tube shields the fastener prior to insertion into tissue.

## Description

### BACKGROUND

### 1. Technical field

The present disclosure relates to a single fire tacker instrument for use in endoscopic or laparoscopic surgery. More particularly, the present disclosure relates to a shielded single fire tacker instrument having a clip to retain a fastener within the instrument until the faster has been deployed into tissue.

### 2. Background Of Related Art

Various surgical procedures require instruments capable of applying fasteners to tissue to form tissue connections or to secure objects to tissue. For example, during hernia repair procedures is often desirable to fasten a mesh to body tissue. In certain hernias, such as direct or indirect inguinal hernias, a part of the intestine protrudes through a defect in the support abdominal wall to form a hernial sac. The defect may be repaired using an open surgery procedure in which a relatively large incision is made and the hernia is closed off outside the abdominal wall by suturing. The mesh is attached with sutures over the opening to provide reinforcement.

Less invasive surgical procedures are currently available to repair a hernia. In laparoscopic procedures, surgery is performed in the abdomen through a small incision while in endoscopic procedures, surgery is performed through narrow endoscopic tubes or cannulas inserted through small incisions in the body. Laparoscopic and endoscopic procedures generally require long and narrow instruments capable of reaching deep within the body and configured to seal with the incision or tube they are inserted through. Additionally, the instruments must be capable of being actuated remotely, that is, from outside the body.

Currently, endoscopic techniques for hernia repair utilize fasteners such as surgical staples or clips, to secure the mesh to the tissue to provide reinforcement in the repair and structure for encouraging tissue regrowth. The staples or clips need to be compressed against the tissue and mesh to secure the two together.

One other type of fastener, and surgical instrument, suited for use in affixing mesh to tissue, during procedures such as hernia repair, is a coil fastener having a helically coiled body portion terminating in a tissue penetrating tip. Unique instruments have been developed to rotate these helically coiled fasteners into tissue. Examples of this type of surgical fasteners and surgical instruments are disclosed in U.S. Patent Nos. 5,258,000 and 5,830,221, the contents of which are incorporated herein by reference.

During certain surgical procedures, it may not be necessary to utilize more than a single fastener to secure the prosthetic to tissue. In these instances, the provision of a multi-fire tacker instrument may not be necessary and may involve unnecessary extra costs.

Thus, there is a need for a tacker instrument which is simple and cost-effective to fire a single fastener into tissue. Additionally there is also a need for a surgical instrument which allows for its actuation mechanism to be reset without affecting the fastener fully or partially applied to the tissue.

### SUMMARY

There is provided a single fire tacker instrument for use in installing a fastener into tissue. The tacker instrument generally includes a handle assembly having a rotator rotatably mounted therein and an elongated tacker assembly extending distally of the handle assembly. The handle assembly includes an actuator, in the form of a trigger, for operating the rotator. The elongated tacker assembly includes a drive rod connected to the rotator. The drive rod terminates in a sharp tissue penetrating tip. The elongated tacker assembly additionally includes a driver configured to engage a fastener. The driver is affixed to the drive rod by means of a pin. The driver includes distally extending tabs configured to engage the head of a fastener.

The elongated tacker assembly additionally includes a spring clip configured to releasably engage the fastener and maintain the fastener in contact with the driver. The spring clip at least partially surrounds the driver. In one embodiment, the spring clip includes flexible inwardly directed projections configured to engage the head of the fastener. The elongated tacker assembly includes a proximal tube configured to engage the handle assembly and support the elongated tacker assembly relative to the handle assembly.

The elongated tacker assembly additionally includes an outer tube mounted for movement relative to the handle assembly. The outer tube terminates in crenellations configured to engage a prosthetic mesh and prevent the mesh from twisting as the fastener is rotated through the mesh into tissue. In one embodiment, the elongated tacker assembly includes a spring affixed to the outer tube to a bias the outer tube in a distal direction.

There is also disclosed an elongated tacker assembly for use with a handle assembly which generally includes an inner tube configured to engage a handle assembly and a drive rod extending through the inner tube and fixed to a rotatable actuator of the handle assembly. The elongated tacker assembly includes a spring clip configured to releasably engage a fastener. The spring clip includes a pair of flexible, inwardly directed projections configured to engage the head of a fastener.

The elongated tacker assembly additionally includes an outer tube mounted for movement relative to an associated handle assembly for actuating various mechanisms associated with the handle assembly. In one embodiment, the elongated tacker assembly includes a spring biasing the outer tube and a distal direction relative to the associated handle assembly.

### DESCRIPTION OF THE DRAWINGS

An embodiment of the presently disclosed tacker instrument is disclosed herein with reference to the drawings, wherein:
FIG. 1 is a side view in partial cross-section of one embodiment of a tacker instrument;
FIG. 2 is a side view, shown in section, of the distal end of the tacker instrument;
FIG. 3 is a perspective view, with parts separated, of the distal end of the tacker instrument;
FIG. 3A is an enlarged view of the distal end of the tacker instrument according to an embodiment of the present disclosure;
FIGS. 4A- 4E are views of a clutch mechanism associated with the tacker instrument; and
FIG. 5 is an enlarged perspective view, partially shown in section, of the distal end of the tacker instrument.

### DETAILED DESCRIPTION

An embodiment of the presently disclosed single fire tack instrument will now be described in detail with reference to the drawings wherein like numerals designate identical or corresponding elements in each of the several views. As is common in the art, the term 'proximal" refers to that part or component closer to the user or operator, i.e. surgeon or physician, while the term "distal" refers to that part or component further away from the user.

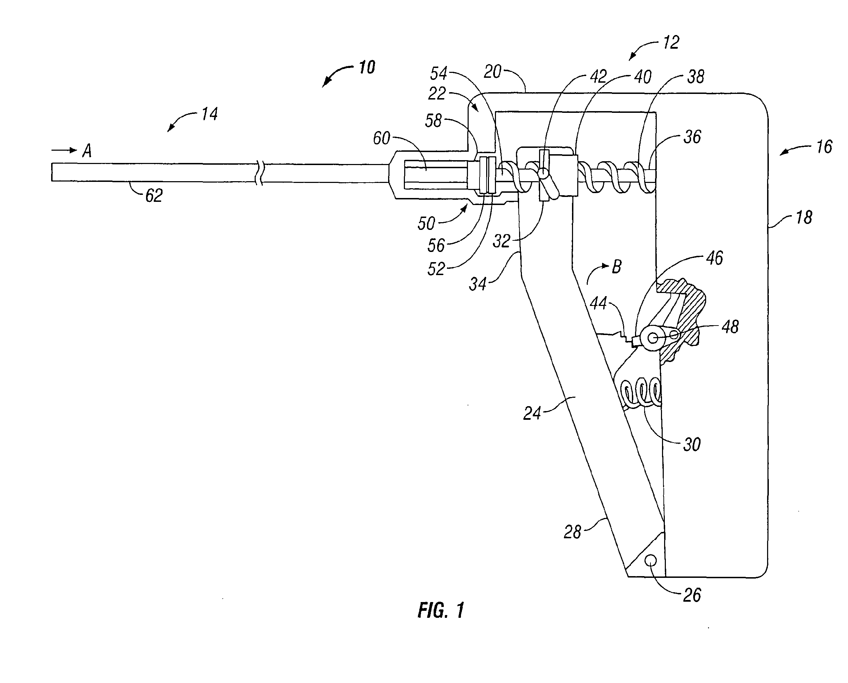

Referring now to FIG. 1, there is disclosed a preferred embodiment of a single fire tack instrument 10 for use in installing a fastener in tissue, such as, for example, securing a prosthetic mesh to tissue during a hernia repair procedure. Tacker instrument 10 generally includes a handle assembly 12 having an elongated tacker assembly 14 extending distally from handle assembly 12. Handle assembly 12 includes a handle housing 16 having a grip portion 18, a bridge portion 20, and a nose portion 22. It should be noted that while handle housing 16 is illustrated in skeletal form so as to view the internal components, in a commercial embodiment, handle housing 16 will be completely sealed to protect the internal components. A trigger 24 is provided on handle housing 16 to actuate tacker instrument 10. Trigger 24 is pivotally attached to grip portion 18 at a pivot point 26 located at a first end 28 of trigger 24. Trigger 24 is biased to a first, spaced apart, position relative to grip portion 18 by a spring 30 positioned between grip portion 18 and trigger 24. Spring 30 allows trigger 24 to return to initial position after trigger 24 has been fully deployed through a first stroke. As noted in more detail hereinbelow, in order to actuate tacker instrument 10, trigger 24 is moved through more than one stroke in order to deploy the fastener associated with tacker instrument 10. A slot 32 is defined in a second end 34 of trigger 24 to facilitate the actuation of tacker instrument 10.

Handle assembly 12 further includes a rotator rod 36 rotatably supported within handle housing 16. Rotator rod 36 includes a helical flute 38 along substantially the length of rotator rod 36. A drive block 40 surrounds rotator rod 36 and is engageable with helical flute 38 so as to rotate rotator rod 36 as drive block 40 is moved in the distal and proximal directions relative to handle housing 16. In order to move drive block 40 in the proximal and distal directions, drive block 40 includes a pin 42 which is configured to ride within slot 32 in trigger 24. Thus, as trigger 24 is depressed, pin 42 moves within slot 32 and draws drive block 40 proximally over helical flute 38 thereby rotating drive rod 36 in a first direction. Once trigger 24 has been fully depressed, it returns to the initial position due to the bias of spring 30 causing drive block 40 to move distally back over helical flute 38 on drive rod 36.

In order to ensure that trigger 24 is fully depressed during actuation, handle assembly 12 includes a ratchet mechanism including a plurality of ratchet teeth 44 formed on trigger 24. Ratchet teeth 44 cooperate with a pawl mechanism 46 which is pivotally attached to grip portion 18 at a pivot point 48. Thus, as trigger 24 is moved proximally relative to handle housing 16, pawl 46 rides on ratchet teeth 44 and prevents trigger 24 from returning to the initial position until trigger 24 has been fully depressed and pawl 46 has cleared ratchet teeth 44. Once pawl 46 has cleared ratchet teeth 44, trigger 24 it is free to return to an initial position as pawl 46 reverses direction and rides back over ratchet teeth 44.

In order to transfer the rotary motion of rotator rod 36 to elongate tacker assembly 14 and thus rotate a fastener out of elongated tacker assembly 14, tacker instrument 10 is provided with a clutch 50 positioned within handle assembly 12. Clutch 50 allows a fastener to be rotated out of tacker instrument 10 in response to actuation of trigger 24 while at the same time allowing trigger 24 to return to an initial position without rotating the fastener in the opposite direction. Specifically, clutch 50 includes a drive plate 52 formed on a distal end of 54 of rotator rod 36. Drive plate 52 is rotated in the same direction as rotator rod 36 upon depression of trigger 24. Clutch 50 additionally includes a transfer plate 56 which engages drive plate 52 as drive plate 52 is rotated in the first direction and disengages from drive plate 52 as drive plate 52 is rotated in an opposite direction in response to release of trigger 24. As shown clutch 50 is rotatably supported with in a journal portion 58 formed in nose portion 22 of handle housing 16.

As shown, elongate tacker assembly 14 includes a drive rod 60 and an outer tube 62. A proximal end 64 of drive rod 60 is connected to transfer plate 56 of clutch 50. Thus, as transfer plate 56 rotates in response to depression of trigger 24, drive rod 60 also rotates in response to depression of trigger 24. Rotation of drive rod 60 rotates a fastener, contained within elongated tacker assembly 14, into tissue in a manner described in more detail hereinbelow. Outer tube 62 of tacker assembly 14 is mounted for longitudinal movement relative to handle assembly 12 for use with alternate handle assembly configurations as discussed in more detail hereinbelow.

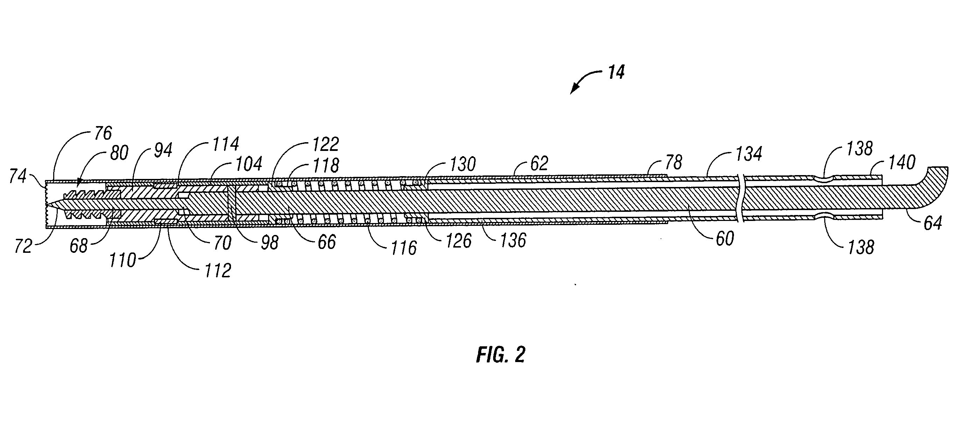

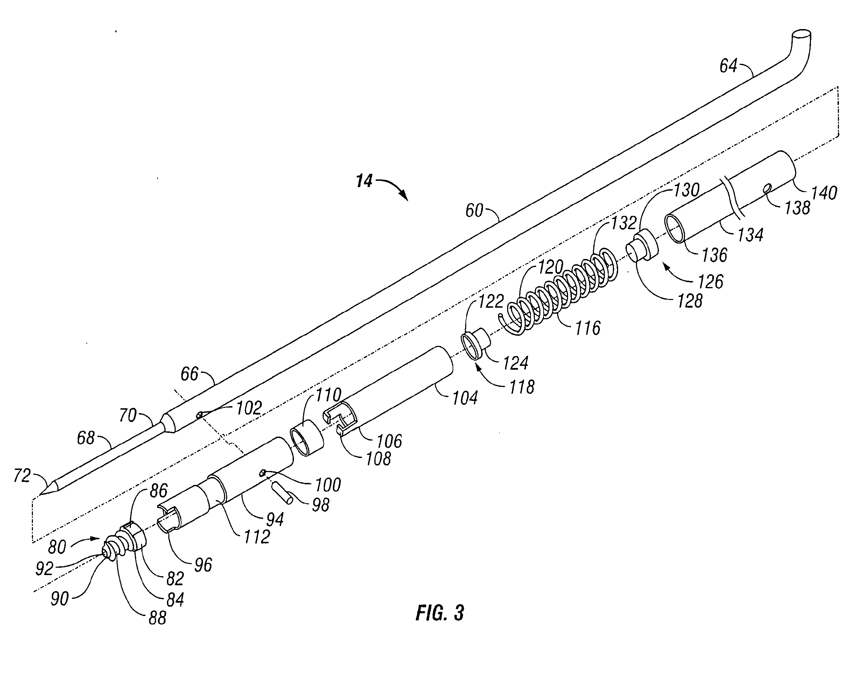

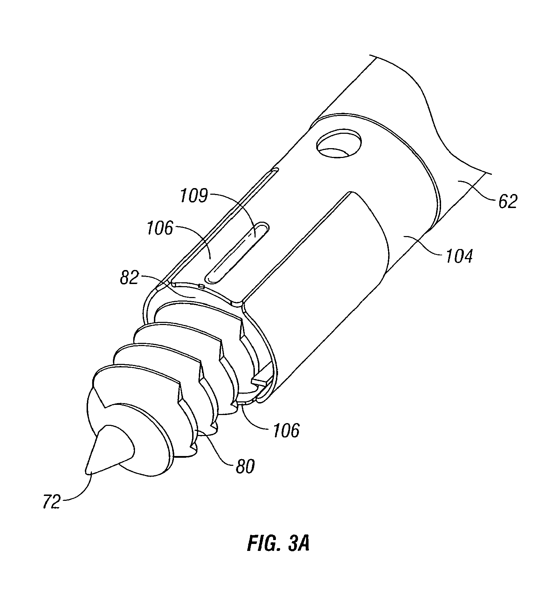

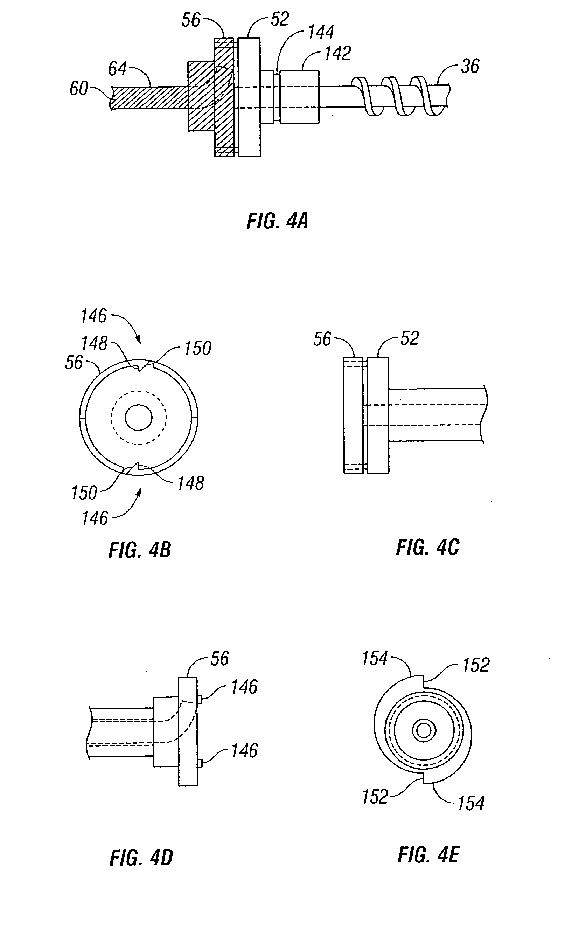

Referring now to FIGS. 2 and 3, elongated tacker assembly 14 includes a drive rod 60 and an outer tube 62. Drive rod 60 includes a generally J-shaped proximal end 64 which is configured to engage transfer plate 56 of clutch 50 (See FIGS. 1, 4A and 4D). Drive rod 60 has a distal end 66 which terminates in a tissue penetrating needle 68 extending distally from distal end 66. Specifically, a proximal end 70 of needle 68 is attached to distal end 66 of drive rod 60. Needle 68 terminates in a sharp distal tip 72. Sharp distal tip 72 is provided to initially pierce tissue, and an associated prosthetic, in advance of the installation of a fastener through the prosthetic and into tissue.

Referring to FIG. 2, outer tube 62 is provided with crenellations 74 at a distal end 76 of outer tube 62. Crenellations 74 assist in securing the prostatic mesh in a stable position against the underlying tissue and inhibit twisting of the mesh as a fastener is driven therethrough. Outer tube 62 has a proximal end 78 which is mounted for movement relative to handle assembly 12. It is envisioned that elongated tacker assembly 14 can be used with alternative handle configurations. Outer tube 62 is movable relative to drive rod 60 and handle assembly 12 so that outer tube 62 may perform additional functions depending upon the particular handle assembly used. For example, in some instances, a handle assembly used with elongate tacker assembly 14 may include various triggers or failsafe devices which are designed to be engaged by outer tube 62 in order to actuate the various triggers or failsafe devices as outer tube 62 is pressed against tissue.

Referring now specifically to FIG. 3, one embodiment of a fastener particularly configured for use with tacker instrument 10 is illustrated. Fastener 80 is of a type typically used for attaching a prosthetic mesh to tissue during a hernia repair procedure. Particular embodiments of fastener 80 are disclosed in US patent application serial number 10/560,879, entitled Multiple Member Interconnect For Surgical Instruments And Absorbable Screw Fastener, filed December 13, 2005, the contents of which are incorporated herein by reference. Fastener 80 generally includes an enlarged head 82 having a distal tissue engaging surface 84. Distal tissue engaging surface 84 is sufficiently large to secure a prosthetic mesh against tissue. Head 82 additionally includes opposed side slots 86 for engagement with a portion of elongated tacker assembly 14 in a manner described in more detail hereinbelow. Fastener 80 further includes an elongate shaft 88, extending distally from head 82, and having a helical thread 90 for rotation into tissue to secure fastener 80 to the tissue. Fastener 80 additionally includes a throughbore 92. Throughbore 92 is configured for receipt of needle 68 so as to guide fastener 80 into tissue.

Referring back to FIGS. 2 and 3, elongate a tacker assembly 14 includes a hollow driver 94 which is configured to engage fastener 80 and rotate fastener 80 through a prosthetic mesh and into tissue. Driver 94 includes a pair of distally extending tabs 96 which are provided to engage slots 86 in fastener 80 so as to rotate fastener 80. In order to rotate driver 94, driver 94 is affixed to drive rod 60 by means of a pin 98. Specifically, driver 94 is provided with a pair of mounting holes 100. Similarly, drive rod 60 is provided with a pair of corresponding mounting holes 102. As shown, driver 94 is fixedly pinned to drive rod 60 by pin 98 so that driver 94 rotates as drive rod 60 is rotated in response to actuation of handle assembly 12. In this manner, actuation of handle assembly 12 serves to rotate fastener 80 through a prosthetic mesh into tissue.

In order to insure that fastener 80 remains secured to driver 94 until fastener 80 is driven into tissue, tacker assembly 14 further includes a spring clip 104 having a pair of flexible legs 106 extending distally therefrom. In an embodiment, a pair of inward projections 108 are provided at the distal end of flexible legs 106 and are configured to engage distal engagement surfaces 84 on fastener 80. Once fastener 80 has been securely inserted into tissue, flexible legs 106 are sufficiently flexible to allow fastener 80 to pull free of inward projections 108. Flexible legs 106 may be oriented parallel with tabs 96 of driver 94 as shown in FIG. 2 or may be oriented perpendicular to tabs 96 as shown in FIG. 3.

FIG. 3A illustrates another embodiment of spring clip 104. In this embodiment, spring clip 104 includes a pair of flexible legs 106, with each flexible leg 106 including a detent 109 thereon. It is envisioned that flexible legs 106 are spring-loaded or otherwise biased towards one another to hold a fastener 80 therebetween. Detents 109 are illustrated inwardly depending from flexible leg 106 and are configured to engage a portion of fastener 80, e.g., enlarged head 82, to frictionally hold fastener 80 in a distal-most position at least partially within outer tube 62. Other shapes and configurations of detent 109 are also contemplated by the present disclosure.

Spring clip 104 is secured to driver 94 by means of an engagement ring 110. Engagement ring 110 sits in a driver groove 112 informed in driver 94. As best shown in FIG. 2, engagement ring also fits within a clip groove 114 formed in spring clip 104. Alternatively, engagement ring 110 may be integrally formed with spring clip 104.

As noted hereinabove, outer tube 62 of the elongated tacker assembly 14 is mounted for longitudinal movement relative to handle assembly 12 as well as the remaining components of tacker assembly 14. Outer tube 62 is biased in a distal direction relative to handle assembly 14 by a spring 116. A hollow distal spring guide 118 is provided at a distal end 120 of spring 116. Distal spring guide 118 includes an outer flange 122 and a proximal cylinder 124. Outer flange 122 is affixed to outer tube 62 to bias outer tube 62 in a distal direction. Proximal cylinder 124 fits in distal end 120 of spring 116 to support spring 116.. Similarly, there is provided a hollow proximal spring guide 126 having a distal cylinder 128 and an outer flange 130. Distal cylinder 128 fits in a distal end 132 of spring 116 to also support spring 116. In the initial position, distal end 76 of outer tube 62 shields fastener 80 from unintentional contact.

Elongate tacker assembly 14 is provided with an inner tube 134 to mount elongated tacker assembly 14 on handle assembly 12. A distal end 136 of inner tube 134 abuts flange 130 of proximal spring guide 126 to provide a base against which spring 116 can compress. A pair of opposed mounting holes 138 is provided at a distal end 140 of inner tube 134 to mount inner tube 134, and thus elongated tacker assembly 14, on handle assembly 12. Handle assembly 12 may be provided with various means of engaging holes 138 in inner tube 134 to secure elongated tacker assembly 14 to handle assembly 12 such as, for example, screws, pins,etc., as well as, detents projecting into the holes to allow elongated tacker assembly 14 to be removable/exchangeable for a new elongated tacker assembly 14.

As best shown in FIG. 2, when elongated tacker assembly 14 is assembled, drive rod 60 extends through inner tube 134 and spring 116. Drive rod 60 is pinned to driver 94 and extends through driver 94 and spring clip 104. Needle 68 of drive rod 60 extends through throughbore 92 formed in fastener 80. Outer tube 62 is affixed to spring 116 and surrounds spring clip 104, spring 60, and inner tube 62.

Referring now to FIGS. 4A- 4E, handle assembly 12 includes a clutch 50 having a drive plate 52 and a transfer plate 56. Drive plate 52 receives the rotary motion of rotation rod 36 and transfers it to transfer plate 56 in order to rotate drive rod 60 in response to actuation of trigger 24. Drive plate 52 includes a proximal end 142 having a support journal 144 which is mounted for rotation within journal portion 58 in nose portion 22 of handle housing 16. As further noted hereinabove, drive plate 50 engages and rotates transfer plate 56 when drive plate 52 is rotated in a first direction and disengages from transfer plate 56 when drive plate 52 is rotated in an opposite direction thereby allowing trigger 24 to return to an initial position without rotation of drive rod 60.

Referring specifically to FIG. 4B, transfer plate 56 is provided with projecting engagement teeth 146 having mating surfaces 148 and override surfaces 150. Likewise, referring to FIG. 4E, drive plate 52 is provided with engagement surfaces 152 and override surfaces 154. Thus, as drive plate 52 is rotated in a first direction, engagement surfaces 152 engage mating surfaces 148 of transfer plate 56 to rotate drive rod 60 in a first direction to thereby insert a fastener into tissue. As trigger 24 is released, drive plate 52 rotates in an opposite direction causing override surfaces 154 of drive plate 52 to ride over, and disengage from, override surfaces 150 of transfer plate 56. This allows trigger 24 to return to an initial position without any reverse rotation of drive rod 60 thereby preventing unthreading of a fastener partially installed in tissue.

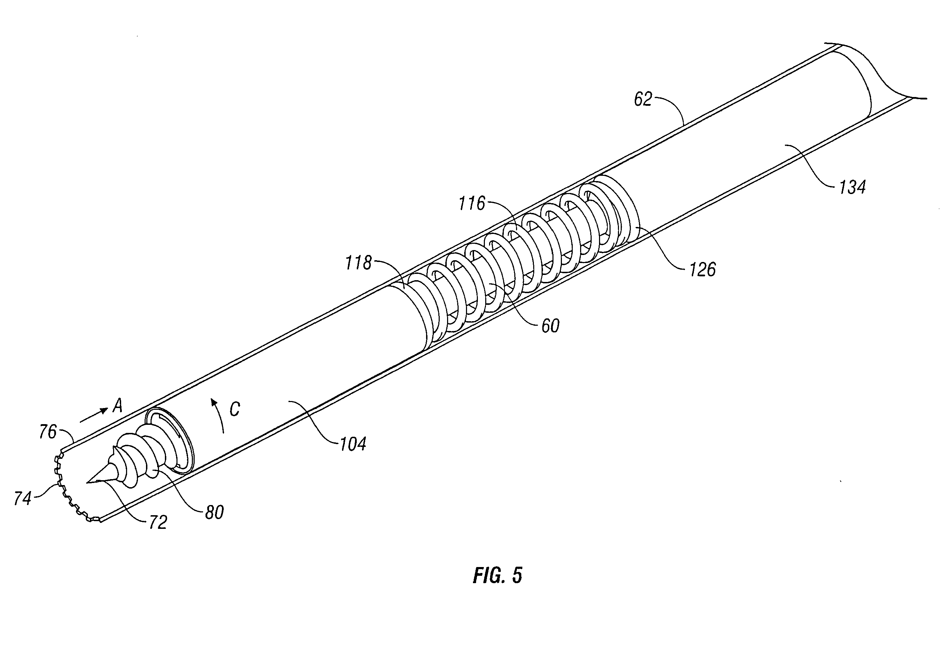

Referring now to FIGS. 1 and 5, the use of tacker instrument 10 to rotate a fastener, such as fastener 80, out of tacker instrument 10 and into tissue will now be described. Initially, tacker instrument 10 is manipulated such that distal end 76 of outer tube 62 is positioned against a desired target area such as, for example, a prosthetic mesh overlying tissue. Tacker instrument 10 is urged towards the tissue such that outer tube 62 is biased proximally in the direction of arrow A against the bias of spring 116. As noted hereinabove, crenellations 74 provided on distal end 76 of outer tube 62 aid in inhibiting the prosthetic mesh from twisting as fastener 80 is driven therethrough. As tacker instrument 10 is urged against the tissue, sharp distal tip 72 of drive rod 60 initially penetrates through the mesh and into tissue thereby forming an initial "pilot hole" into which fastener 80 can be rotated.

Once sharp distal tip 72 has penetrated tissue, trigger 24 can be squeezed in the direction of arrow B so as to cause rotation of rotator rod 36. As rotator rod 36 is rotated, drive plate 52 rotates transfer plate 56 and thus drive rod 60 in the direction of arrow C (FIG. 5). Rotation of drive rod 60 rotates driver 94, along with spring clip 104, to rotate fastener 80 through a prosthetic mesh and into tissue. As noted hereinabove, the interaction of pawl 46 with ratchet teeth 44 on trigger 24 prevent return of trigger 24 until such time as trigger 24 has been completely depressed.

In a particular embodiment, it may be necessary to actuate trigger 20 more than once to fully rotate fastener 80 into tissue. In such instance, as trigger 24 is returned to an initial position, drive plate 52 disengages from transfer plate 56 so that fastener 80 is not counterrotated back out of tissue. Thereafter, trigger 24 can again be squeezed to finish rotating fastener 80 into tissue. Once fastener 80 has been fully seated through the prosthetic mesh and into tissue, distal engagement surfaces 84 of fastener 80 secure the mesh to tissue. Flexible legs 106 disengage from engagement surfaces 84 thereby releasing fastener 80 from tacker instrument 10.

It will be understood that various modifications may be made to the embodiments disclosed herein. For example, alternative hollow fasteners having differing thread configurations may be used with the disclosed single fire tacker instrument. Further, as noted hereinabove, various alternative handle assemblies may be used with the disclosed elongated tacker assembly. Therefore, the above description should not be construed as limiting, but merely as exemplifications of particular embodiments. Those skilled in the art will envision other modifications within the scope and spirit of the claims appended hereto.

## Claims

1. A single fire tacker instrument for installing a fastener in tissue comprising:
a handle assembly having a rotator rotatably mounted therein;
an actuator of associated with a handle assembly for rotating the rotator; and
an elongated tacker assembly affixed to the handle assembly and including a drive rod and a driver affixed to the drive rod, the driver configured to engage a fastener, said drive rod including tissue penetrating structure.

2. The single fire tacker instrument as recited in claim 1, wherein the driver includes distally extending tabs configured to engage slots in the head of a fastener.

3. The single fire tacker instrument as recited in claim 1 or 2, wherein the tissue penetrating structure is a sharp tissue penetrating needle tip.

4. The single fire tacker instrument as recited in claim 1, 2 or 3, wherein the driver is affixed to the drive rod by means of a pin.

5. The single fire tacker instrument as recited in any one of claims 1 to 4, wherein the elongated tacker assembly includes a spring clip configured to releasably engage the fastener.

6. The single fire tacker instrument as recited in claim 5, wherein the spring clip at least partially surrounds the driver.

7. The single fire tacker instrument as recited in claim 5 or 6, wherein the spring clip includes a pair of inward projections configured to releasably engage the head of a fastener.

8. The single fire tacker instrument as recited in any one of claims 1 to 7, wherein the elongated tacker assembly includes a proximal tube configured to engage the handle assembly.

9. The single fire tacker instrument as recited in claim 8, wherein the elongated tacker assembly includes an outer tube mounted for movement relative to the handle assembly.

10. The single fire tacker instrument as recited in claim 9, wherein the outer tube terminates in crenellations configured to engage a prosthetic mesh applied over tissue.

11. The single fire tacker instrument as recited in claim 9 or 10, wherein the elongated tacker assembly includes a spring to bias the outer tube in a distal position relative to the handle assembly.

12. The single fire tacker instrument as recited in claim 11, wherein the spring surrounds the drive rod and is affixed to the outer tube.

13. An elongated tacker assembly configured for use with a handle assembly to install a fastener into tissue comprising:
an inner tube configured to engage a handle assembly;
a drive rod extending through the inner tube and a fixed to a rotatable actuator of the handle assembly; and
a driver affixed to the drive rod, the driver configured to engage a fastener and rotate the fastener into tissue in response to actuation of the actuator of the handle assembly.

14. The elongated tacker assembly as recited in claim 13, wherein the elongated tacker assembly includes a spring lip configured to releasably engage a fastener.

15. The elongated tacker assembly as recited in claim 14, wherein the spring clip surrounds the driver.

16. The elongated tacker assembly as recited in claim 14 or 15, wherein the spring clip includes a pair of flexible inwardly directed projections configured to engage the head of a fastener.

17. The elongated tacker assembly as recited in claim 13, 14, 15 or 16, wherein the elongated tacker assembly includes an outer tube mounted for movement relative to an associated handle assembly.

18. The elongated tacker assembly as recited in claim 17, wherein the elongated tacker assembly includes a spring biasing the outer tube in a distal direction relative to an associated handle assembly.
